# EUROPEAN PATENT SPECIFICATION

(11) **EP 1 748 705 B1**
(45) Date of publication and mention of the grant of the patent: **13.10.2010**
(21) Application number: 05738192.3
(22) Date of filing: 10.05.2005
(51) Int. Cl.: A23L 1/30, A23L 2/38, A23L 1/164, A23K 1/16, A23L 1/29

(54) **USE OF BETA-CRYPTOXANTHIN**
VERWENDUNG VON BETA-CRYPTOXANTHIN
UTILISATION DE $G(B)-CRYPTOXANTHINE

(30) Priority: 18.05.2004 EP 04011743
(43) Date of publication of application: 07.02.2007
(73) Proprietor: DSM IP Assets B.V., 6411 TE Heerlen (NL)
(72) Inventor: EICHINGER, Anne, F-68200 Mulhouse (FR); GORALCZYK, Regina, 79639 Grenzach-Wyhlen (DE); WERTZ, Karin, 79618 Rheinfelden (DE); WYSS, Adrian, CH-4052 Basel (CH)
(74) Representative: Steck, Melanie
(86) International application number: PCT/EP2005/005030
(87) International publication number: WO 2005/110122

(56) References cited:
- US-A1- 2003 118 672
- US-B1- 6 582 721
- DATABASE EMBASE [Online] ELSEVIER SCIENCE PUBLISHERS, AMSTERDAM, NL; 2004, YAMAGUCHI M: "[beta]-Cryptoxanthin stimulates bone formation and inhibits bone resorption in tissue culture in vitro" XP002334583 Database accession no. EMB-2004099198 & MOLECULAR AND CELLULAR BIOCHEMISTRY 2004 NETHERLANDS, vol. 258, no. 1-2, 2004, pages 137-144, ISSN: 0300-8177

## Description

The present invention relates to the use of β-cryptoxanthin in human and in animals. More particularly the present invention relates to use of β-cryptoxanthin in the manufacture of a composition for promoting an increased protein formation and/or prevention of loss of proteins. In a further aspect, the present invention relates to a method for promoting an increased protein formation and/or prevention of loss of protein in human or an animal which comprises administering to said human or animal an effective amount of β-cryptoxanthin.

US 2003/0118672 (NutraStar) describes formulations for treating inflammation which contain a stabilized rice bran derivative and a fortification agent such as glucosamine derivatives, methylsulfonylmethane, yucca concentrate or grape seed extract.

The term β-cryptoxanthin as used herein comprises β-cryptoxanthin either from natural source or synthetically prepared β-cryptoxanthin. β-Cryptoxanthin (more specifically, (all-E) β-cryptoxanthin) from natural source may contain β-cryptoxanthin esters with saturated and unsaturated fatty acids, (mainly laurate, myristate, palmitate, stearate, linolate,) as well as the isomers (preferably 7', 9', 11' and 13' β-cryptoxanthin) which are included also for use in the present invention. In a preferred aspect, synthetically prepared (all-E)-β-cryptoxanthin is used for the purposes of the invention.

The present invention especially relates to the use of β-cryptoxanthin in the manufacture of a composition for enhancement and increase of protein synthesis in tissue (i.e. liver, skin, kidney, muscle etc.) of humans and animals in all conditions where an increased protein formation in the body is needed or desirable, e.g., in adverse health conditions, such as tumor cachexia, eating disorders (e.g., bulimia and anorexia), chronic diseases, such as chronic heart failure, chronic obstructive pulmonary disease, chronic bowel diseases (e.g. Crohn's disease), chronic degenerative diseases e.g., osteoporosis, rheumatoid arthritis, osteoarthritis; as well as to generally foster growth and development, in sports and workout, convalescence, and during aging for prevention and treatment of sarcopenia. In addition, the present invention relates to the use of β-cryptoxanthin in the manufacture of a composition enhancing performance and promoting growth of farm animals, pets and competitive sport animals. The invention also comprises the use of β-cryptoxanthin as an agent in the treatment of conditions where an increased protein formation in the body is needed or desirable.

In particular, β-cryptoxanthin can find use in accordance with the present invention to enhance and/or to potentiate the protein synthesis in case of syndromes and pathologies inducing protein wasting, or in the prophylactic supplementation of healthy subjects. Further examples of adverse health conditions where β-cryptoxanthin may find use in accordance with the invention are states of infections with the Human Immunodeficiency Virus when patients show the first signs of the Acquired ImmunoDeficiency Syndrome (AIDS), cases of recent transplants, and chronic alcohol abuse. β-Cryptoxanthin may also be used in bedridden people for prevention and treatment of immobilization induced muscle loss, or in people suffering from other long lasting infectious diseases, like hepatitis, Epstein-Barr Virus and Varicella zoster infections. Further, β-cryptoxanthin may be used in accordance with the invention in the improvement of the general health status of convalescent patients, e.g., after second or third degree bruns, myocardial infarction, and organ transplantation. It has been found also that the promotion of protein formation by β-cryptoxanthin in accordance with the present invention is even more effective when bile salts (e.g., sodium cholate, glycocholate, taurocholate, glycodeoxycholate) are present in the gut. Thus, in another aspect of the invention, β-cryptoxanthin is used in combination with choleretic stimulants, or by addition of bile salts to the diet. Examples of choleretic stimulants for use in the present invention are coffee, green or black tea, herbs such as camomile , rosemary, mint, mate, milk thistle, lavender, fennel, artichoke as teas or extracts thereof, or polyunsaturated fatty acids, suitably as capsules. In still another aspect of the invention, β-cryptoxanthin is used in combination with vitamin C and/or vitamin E. Further, β-cryptoxanthin may be used for the purposes of this invention in combination with other carotenoids (e.g., β-carotene, lutein, zeaxanthin, lycopene, astaxanthin, canthaxanthin and/or apocarotenals), genistein, resveratrol, and (-)-epigallocatechin gallate (EGCG).

In accordance with the present invention, β-cryptoxanthin is suitably administered in dosages up to about 50 mg/day, more particularly, from about 100 µg /day to about 30 mg/day, especially from about 1 mg /day to about 10 mg/day for a human adult of about 70 kg body weight. If added as a supplement to animal feed, β-cryptoxanthin may be used in an amount to provide from about 100 to about 1000 ppm of β-cyptoxanthin in the final feed composition. If choleretic stimulants are co-administered, the amount of these may be in case of teas from about 1 to 5 cups per day, or in case of extracts, about 10mg to about 500mg per day for a human adult. If vitamin vitamin E is co-administered, the dosage of these is suitably from about from about 15 to about 500 mg vitamin E per day for a human adult. If vitamin vitamin C is co-administered, the dosage of that is suitably from about from about 50 to about 500 mg per day for a human adult.

For the purposes of the invention, β-cryptoxanthin is suitably provided in compositions for enteral application which may be solid or liquid galenical formulations, dietary compositions, animal feed or feed premixes for animals. Examples of solid galenical formulations are tablets, capsules (e.g. hard or soft shell gelatin capsules), pills, sachets, powders, granules and the like which contain the active ingredient together with conventional galenical carriers. Any conventional carrier material can be used. The carrier material can be organic or inorganic inert carrier material suitable for oral administration. Suitable carriers include water, gelatin, gum arabic, lactose, starch, magnesium stearate, talc, vegetable oils, and the like. Additionally additives such as flavouring agents, preservatives, stabilizers, emulsifying agents, buffers and the like may be added in accordance with accepted practices of pharmaceutical compounding. Additional active ingredients for co-administration with β-cryptoxanthin may administered, together with β-cryptoxanthin in a single composition, or may be administered in individual dosage units. Dietary compositions comprising β-cryptoxanthin can be beverages, instant beverages, or food/feed supplements.

As stated earlier, β-cryptoxanthin may be used in accordance with the present invention together with vitamin C or/and vitamin E, as well as in combination with other carotenoids (e.g., β-carotene, lutein, zeaxanthin, lycopene, astaxanthin, canthaxanthin, apocarotenals), genistein, resveratrol, and/or EGCG, which may be administered simultaneously with β-cryptoxanthin or separately. Furthermore, β-cryptoxanthin can be used in accordance with the present invention in combination with other vitamins and/or minerals conventionally used as food supplement, such as vitamins of the B group, and minerals containing Ca, Fe, P, Mg, Zn etc. A typical solid galenical formulation contains per dosage unit from about 1 µg to about 50 mg β-cryptoxanthin and, optionally, from about 15 mg (22 IU) to about 500 mg (750 IU) vitamin E. A typical liquid galenical formulation may contain, per ml, from about 10 ng to about 50 µg β-cryptoxanthin and, optionally, from about 10 mg (15 IU) to about 50 mg (75 IU) vitamin E. A typical dietary composition may contain from about 0.1 µg to about 5 mg β-cryptoxanthin and, optionally from about 1.5 mg (2.25 IU) to about 30 mg (45 IU) vitamin E per g of the total composition.

The efficacy of β-cryptoxanthin in promoting increased protein formation is evident from findings that upon administration of β-cryptoxanthin, genes involved in protein synthesis in skin, liver, kidney and spleen were up-regulated. In a six weeks study, β-cryptoxanthin beadlets were mixed into the diet of SKH-1 mice to provide 1200 ppm β-cryptoxanthin in the feed. Further, 0.125 g of sodium cholate per 100 g of feed was added to facilitate the uptake of β-cryptoxanthin. The average β-cryptoxanthin intake was 6.5 mg pro mouse and pro day. After six weeks, the effect of this dose on the overall gene expression was studied. β-cryptoxanthin-mediated gene expression modifications were assessed by GeneChip^{®} DNA microarray technology. Total RNA was extracted from kidney, liver, skin and spleen using the TRlzol^{®} method according to the manufacturer protocol. Reverse Transcription was performed with 10 µg total RNA followed by second strand synthesis of cDNA. The cDNA was then transcribed into multiple cRNA copies and labelled by biotin-coupled nucleotides and fragmented. 10 µg of labelled and fragmented cRNA were hybridized on Affymetrix Mice Genome 430A microarrays as described in the GeneChip^{®} Expression Analysis Technical Manual (Affymetrix, Oxford, UK). For each β-cryptoxanthin-treated tissue, five replicates were performed and compared to six replicates of untreated control mice. After washing and staining procedures, the signals were amplified with a biotinylated anti-streptavidin antibody using the GeneChip^{®} Fluidics Station. Subsequently, the microarrays were subjected to laser scanning and the hybridization signals were analyzed with Genedata Expressionist^{®} and Phylosopher^{®} software packages (Genedata, Basel, Switzerland). The primary goal was to identify genes with significant differences in expression between the control and the treated groups. Comparisons were made by computing the expression fold difference for each gene and listing those that showed greater than 1.20-fold increases or decreases in activity. Statistical analysis of GeneChip^{®} arrays was performed using the Welch-test to identify compound-responsive genes in skin, kidney, liver and spleen. Statistical significance for the DNA microarrays was accepted at p-values < 0.01.Genedata Phylosopher^{®} software was used to assign the Affymetrix probe sequences to pathways and functional categories.

The results of this study focusing on the upregulation of genes which are involved in protein synthesis pathways are shown in Table 1.

Up-regulation of ribosomal proteins gene expression can be correlated with an increase of the overall protein synthesis insofar as ribosomes represent a highly efficient molecular machine catalizing protein synthesis (Al-Karadaghi et al., Prog. Biophys. Mol Biol, 2000, 73(2-4):167-93). Ribosomes are composed of two subunits, the large and the small one. Whereas the small subunit includes in bacteria 21 ribosomal proteins (S1-S21), the large subunit consists of 36 ribosomal proteins (L1-L36) (Wittmann HG., Annu Rev Biochem, 1982, 51:155-83). More recent data indicated that protein synthesis is accomplished by ribosomes acting in concert with tRNA and accessory factors to "translate" the genetic information contained in mRNA (Preiss et al., Bioessays, 2003, 25(12):1200-11). mRNA translation into protein can be divided into three successive phases : initiation, elongation and termination. Briefly, the initiation phase consists in the assembly of a ribosome with the initiator Met-tRNA at the start codon of the mRNA. The protein synthesis really begins during the elongation phase. When the ribosome reaches the stop codon, this terminates the protein synthesis by releasing the polypeptide, and probably the ribosome from the mRNA (Preiss et al., supra).

Thus the ribosome can be considered as a crucial component allowing protein synthesis to occur.
β-Cyptoxanthin enhances protein formation in muscle cell cultures

C2C12 cells from American Type Culture Collection (ATCC, CRL-1772).
The mouse myoblast cell line C2C12 was seeded into 6 cm² cell culture dishes at a density 2x10⁵ cells /per well. Cells were cultivated in the presence of Dulbecco's Minimum Essential Medium (DMEM) containing a final concentration of 10%fetal calf serum, 4500 mg/L glucose, 100 IU/ml Penicillin & 100 µg/ml Streptomycin 2 mM L-Glutamine and 1 mM sodium pyruvate. One day post seeding, cells were supplemented with fresh medium containing β-cryptoxanthin with a final concentration of 0.25µM or 1 µM or vehicle (tetrahydrofurane). β-cryptoxanthin stock solution was prepared in tetrahydrofurane and the solvent concentration in the media was kept constant at 0.14% for all treatment conditions. Cultivation in medium containing β-cryptoxanthin followed up for a total of 7 consecutive days. The medium was changed every second day. On each day, cells from one well per treatment group were scraped into 0.4 ml of a lysis buffer consisting of 20 mM Tris pH 7.5, NaCl 150 mM, EDTA 1 mM, Nonidet 1%, proteinase inhibitors (Roche Molecular Systems, Mannheim Germany) and sonicated.

Aliquots of 10 µl of this solution were used for total protein, and 2 µl for total DNA analysis. After centrifugation for 5 min at 10000 g, supernatants were used for protein and DNA analysis in the soluble fraction.

Protein was determined using the BCA Protein Assay Reagent (Pierce, Rockford, USA). The Pico-green Assay (Molecular Probes, Leiden, The Netherlands) was used for analysis of DNA content.

The amount of protein was calculated as µg/well or in relation to the amount of cells present in the well, as indirectly determined by the amount of DNA/well. The results are shown in Tables 2 to 4.

**Table 2**

| total protein µg/well after 1-7 days [d] in culture | | | | | | | |
|---|---|---|---|---|---|---|---|
| [d] | 1 | 2 | 3 | 4 | 5 | 6 | 7 |
| Vehicle control 0.25 µM | 707.2 | 674.8 | 707.2 | 903.6 | 1086 | 1082.4 | 950 |
| β-cryptoxanthin 1 µM | 703.6 | 753.6 | 771.2 | 1136 | 1314.4 | 1411.2 | 1257.2 |
| β-cryptoxanthin | 750 | 757.2 | 750 | - | 1236 | 1232.4 | 1193.2 |

**Table 3**

| soluble protein µg/well after 1-7 days [d] in culture | | | | | | | |
|---|---|---|---|---|---|---|---|
| [d] | 1 | 2 | 3 | 4 | 5 | 6 | 7 |
| Vehicle control 0.25 µM | 671 | 540 | 619 | 760 | 940 | 943 | 860 |
| β-cryptoxanthin 1 µM | 536 | 592 | 626 | 915 | 1091 | 1267 | 1284 |
| β-cryptoxanthin | 574 | 547 | 609 | 857 | 1126 | 1146 | 1118 |

**Table 4**

| µg soluble protein/ µg DNA after 1-7 days [d] in culture | | | | | | | |
|---|---|---|---|---|---|---|---|
| [d] | 1 | 2 | 3 | 4 | 5 | 6 | 7 |
| Vehicle control 0.25 µM | 299 | 228 | 256 | 339 | 429 | 471 | 483 |
| β-cryptoxanthin 1 µM | 227 | 244 | 274 | 406 | 505 | 713 | 693 |
| β-cryptoxanthin | 246 | 231 | 265 | 382 | 539 | 624 | 644 |

The data from the above Tables demonstrate that β-cryptoxanthin showed an approx. 1.4 fold increase in total protein (Table 2), an increase in soluble protein (Table 3) and an increase in the specific amount of protein/ µg DNA (Table 4) at the concentration of 0.25 µM. The effect plateaued at 1 µM.

Accordingly, the above data show that β-cryptoxanthin is an effective enhancer of protein formation in muscle cells.

The invention is illustrated further by the Examples which follow.

### Example 1

Beadlets comprising the ingredients as indicated (wt.-%) can be prepared using conventional technology:

| | |
|---|---|
| β-Cryptoxanthin | 5.0 |
| Gelatine 140 Bloom | 32.5 |
| Sucrose | 31.5 |
| Na-ascorbat | 2.0 |
| Ascorbyl palmitate | 2.0 |
| dl-alpha tocopherol | 1.0 |
| Fluid corn starch | 25 |

The beadlets are directly mixed into animal feed at concentrations up to 1200 mg of β-cryptoxanthin/kg of feed.

### Example 2

A tablet is formulated to contain :

| | |
|---|---|
| Active ingredients: | |
| β-cryptoxanthin | 15 mg |
| dl-alpha tocopherol | 300 mg |

| | |
|---|---|
| Excipients : | |
| lactose powder | |
| saccharose | |
| magnesium stearate | |
| gelatin | ad 500 mg |

For the treatment of chronic diseases (chronic renal failure, chronic heart insufficiency, chronic obstructive pulmonary disease, tumor cachexia, sarcopenia) one tablet per day may be administered to a human adult.

### Example 3

A capsule is prepared containing the following ingredients:

| | |
|---|---|
| Active ingredients : | |
| β-cryptoxanthin | 5 mg |
| dl-alpha tocopherol | 100 mg |

| | |
|---|---|
| Excipients : | |
| gelatin | |
| lactose | |
| magnesium stearate | |
| rice starch | |
| glycerol palmitostearate | ad 300 mg |

For the treatment of chronic diseases (chronic renal failure, chronic heart insufficiency, chronic obstructive pulmonary disease, tumor cachexia, sarcopenia) one to three capsules per day may be administered to a human adult.

### Example 4

An Infant Formula prepared with the following components may contain per 100g :

| | |
|---|---|
| β**-**cryptoxanthin | 1 mg |
| Total fat | 3.7g |
| Sodium | 19 mg |
| Potassium | 76 mg |
| Total carbohydrate | 7.7 g |
| Protein | 1.5g |

Vitamins and minerals :
- vitamin A 210 IU
- vitamin C 8.4 mg
- vitamin D 42 IU
- vitamin E 1.5 IU
- vitamin K 5.6 µg
- thiamin (B1) 56.3 µg
- riboflavin (B2) 63.4 µg
- niacin (B3) 704.2 µg
- vitamin B6 42.2 µg
- folate, folic acid, folacin 11.3 µg
- vitamin B12 0.2 µg
- biotin 2.1 µg
- pantothenic acid 352.1 mg
- calcium 54.9 mg
- iron 1.3 mg
- phosphorus 37.3 mg
- iodine 7 µg
- magnesium 5.6 mg
- zinc 0.7 mg
- selenium 2 µg
- copper 52.8 µg
- manganese 10.6 µg
- chloride 44.4 mg
- potassium 76 mg
- choline 8.4 mg
- inositol 4.2 mg
- linoleic acid 605.6 mg
- water 94.4 g

After mixing of all the mentioned components, the infant formula was lyophilized to become a powder. Infants from 1 to 12 months may receive10 g of infant formula per kg body weight per day.

### Example 5

An energy drink of 250 ml may contain :

| | |
|---|---|
| β-cryptoxanthin | 5 mg |
| proteins | 3.9 g |
| lipids | 14.3 g |
| carbohydrates | 71 g |
| H₂O ad 250 ml | |

Vitamin and minerals :
- vitamin E 5 mg
- vitamin B1 1.2 mg
- vitamin B2 1.2 mg
- vitamin B6 1.2 mg
- vitamin C 45 mg
- niacin 9 µg
- sodium 190 mg
- potassium 180 mg
- calcium 70 mg
- phosphor 120 mg
- magnesium 30 mg
- iron 1.3 mg
- zinc 1.6 mg

Teenagers and young people typically consume 100 - 500 ml of energy drink per day.

### Example 6

An energy bar of 25 g may contain :

| | |
|---|---|
| β-cryptoxanthin | 2.5 mg |
| proteins | 1 g |
| lipids | 3.5 g |
| carbohydrates | 16.5 g |
| fibres | 3 g |

Vitamins and minerals :
- vitamin E 5 mg
- vitamin B1 1.2 mg
- vitamin B2 1.2 mg
- vitamin B6 1.2 mg
- vitamin C 45 mg
- niacin 11 mg
- sodium 190 mg
- potassium 180 mg
- chloride 210 mg
- calcium 70 mg
- phosphor 120 mg
- magnesium 30 mg
- iron 1.3 mg
- zinc 1.6 mg

Teenagers and young people typically consume 1-3 energy bars per day.

### Example 7

A sport drink of 250 ml contains :

| | |
|---|---|
| β-cryptoxanthin | 10 mg |
| proteins | 3.9 g |
| lipids | 14.3 g |
| carbohydrates | 71 g |
| H₂O ad 250 ml | |

Vitamins and minerals :
- vitamin E 5 mg
- vitamin B1 1.2 mg
- vitamin B2 1.2 mg
- vitamin B6 1.2 mg
- vitamin C 45 mg
- niacin 9 mg
- sodium 190 mg
- potassium 180 mg
- calcium 70 mg
- phosphor 120 mg
- magnesium 30 mg
- iron 1.3 mg
- zinc 1.6 mg

An athlete or bodybuilder weighing 90 kg typically consumes 250 to 500 ml of sports drink daily. This drink is also suitable for elderly people to prevent or ameliorate sarcopenia.

### Example 8

A feed premix for poultry contains per kg

| | |
|---|---|
| Vitamin A (Rovimix^{®} A 500) | 2 g |
| Vitamin D3 (Rovimix^{®} D3 500) | 1 g |
| Vitamin E (Rovimix^{®} E 50 Ads) | 4 g |
| Vitamin B12 (B12 1%) | 0.1g |
| Vitamin B3 (Rovimix^{®} B2 80-SD) | 0.5 g |
| Niacin (Rovimix^{®} Niacin) | 0.1 g |
| Calpan (Rovimix^{®} Calpan) | 0.05 g |
| Folic acid (Rovimix^{®} Folic 80 SD) | 2 mg |
| Vitamin B6 (Rovimix^{®} 86) | 8 mg |
| Vitamin B1 (Rovimix^{®} B1) | 4 mg |
| Vitamin C | 0.2 g |
| Carotenoids (Carophyll^{®} Red) | 2 g |
| β-cryptoxanthin beadlet formulation 5 % (see Example 1) | 2 g |
| Choline chloride 50% | 150 g |
| Mn(IV)oxide | 30 g |
| Zn oxide | 6 g |
| Fe(II)sulfate monohydrate | 10 g |
| Cu(II)oxide | 1 g |
| Co(II)sulfate | 0.1g |
| | |
| Carrier | |
| (Limestone, rice hulls, wheat middlings) | 792 g |
| | |
| Total | 1000 g |

Animal feed is supplemented with 1 to 30g feed premix per kg feed.

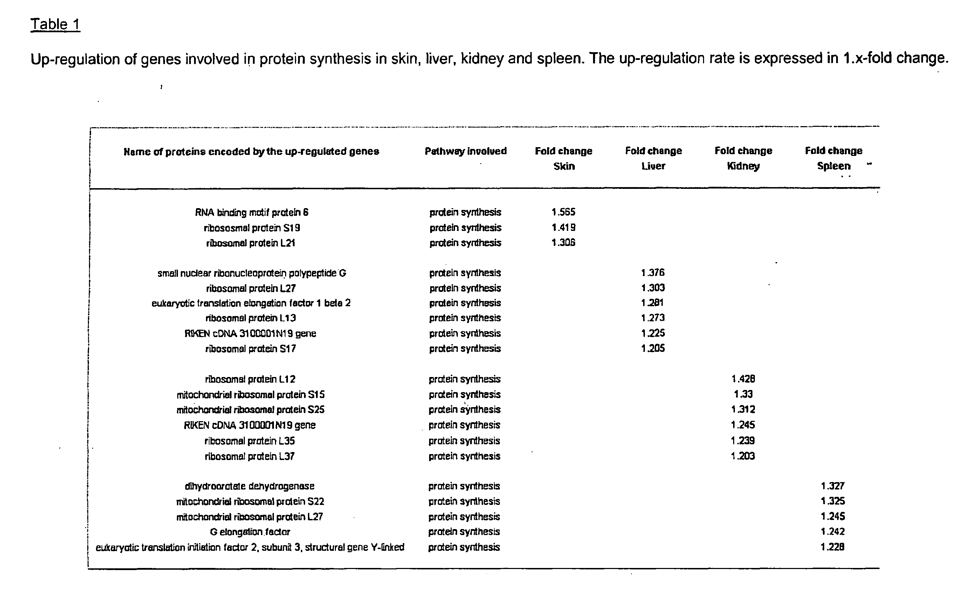

## Claims

1. The non-therapeutic use of β-cryproxanthin for promoting an increased protein formation in a human or an animal.

2. The use as in Claim 1 wherein the increased protein formation fosters growth and development, or promotes increased protein formation in sports and workout activities.

3. The use as in Claim 2 wherein the increased protein formation is for promoting growth in farm animals and pets, and competitive sports animals.

4. The use as in any of Claims 1-3 wherein the β-cryproxanthin is present in a dietary composition.

5. The use as in Claim 4 wherein the dietary composition is a beverage, an instant beverage, a food, or a food supplement.

6. A method for promoting an increased protein formation to enhance performance of compeptive sports animals which comprises administering to said animal an effective amount of β-cryproxanthin.

7. The use as in Claim 4 wherein a bile salt or choleretic stimulant is co-administered.

8. The use of β-cryproxanthin in the manufacture of a medicament for the treatment of an adverse health condition, wherein the adverse health condition is selected from the group consisting of:
a chronic disease, tumor cachexia, an eating disorder, a viral infection, after a second or third degree bum, after a myocardial infarction, after an organ transplant,
immobilization after skeletal injury or surgery, or prevention and/or treatment of
sarcopenia during ageing
by promoting increased protein formation and/or prevention of loss of protein.

## Patentansprüche

1. Nichttherapeutische Verwendung von β-Cryproxanthin zum Fördern einer erhöhten Proteinbildung in einem Menschen oder einem Tier.

2. Verwendung gemäß Anspruch 1, wobei die erhöhte Proteinbildung Wachstum und Entwicklung fördert oder erhöhte Proteinbildung bei Sport- und Trainingsaktivitäten fördert.

3. Verwendung gemäß Anspruch 2, wobei die erhöhte Proteinbildung zum Fördern von Wachstum bei Nutztieren, Haustieren und Sportwettkampftieren ist.

4. Verwendung gemäß einem der Ansprüche 1-3, wobei das β-Cryproxanthin in einer Ernährungszusammensetzung vorhanden ist.

5. Verwendung gemäß Anspruch 4, wobei die Ernährungszusammensetzung ein Getränk, ein Instantgetränk, ein Nahrungsmittel oder ein Nahrungsergänzungsmittel ist.

6. Verfahren zum Fördern einer erhöhten Proteinbildung zum Verbessern der Leistung von Sportwettkampftieren, umfassend das Verabreichen einer

7. Verwendung gemäß Anspruch 4, wobei in Kombination ein Gallensalz oder ein choleretisch stimulierendes Mittel verabreicht wird.

8. Verwendung von β-Cryproxanthin bei der Herstellung eines Medikaments für die Behandlung eines ungünstigen Gesundheitszustands, wobei der ungünstige Gesundheitszustand ausgewählt ist aus der Gruppe, bestehend aus:
einer chronischen Erkrankung, Tumorkachexie, einer Essstörung, einer viralen Infektion, nach einer Verbrennung zweiten oder dritten Grades, nach einem Herzinfarkt, nach einer Organtransplantation, Immobilisierung nach einer Skelettverletzung oder -operation, und Vorbeugung und/oder Behandlung von Sarkopenie beim Altern,
durch Fördern erhöhter Proteinbildung und/oder Vorbeugen von Proteinverlust.

## Revendications

1. Utilisation non thérapeutique de β-cryproxanthine pour stimuler une formation de protéine augmentée chez un humain ou un animal.

2. Utilisation selon la revendication 1 **caractérisée en ce que** la formation de protéine augmentée favorise la croissance et le développement, ou stimule une formation de protéine augmentée dans le sport et les activités d'entraînement.

3. Utilisation selon la revendication 2 **caractérisé en ce que** la formation de protéine augmentée est destinée à stimuler la croissance chez des animaux d'élevage et des animaux domestiques, et des animaux de sport de compétition.

4. Utilisation selon l'une quelconque des revendications 1 à 3 **caractérisée en ce que** la β-cryproxanthine est présente dans une composition diététique.

5. Utilisation selon la revendication 4 **caractérisée en ce que** la composition diététique est une boisson, une boisson instantanée, un aliment, ou un supplément alimentaire.

6. Procédé pour stimuler une formation de protéine
augmentée afin d'augmenter les performances d'animaux
de sport de compétition qui comprend l'administration audit animai d'une quantité efficace de β-cryproxanthine.

7. Utilisation selon la revendication 4 **caractérisée en ce qu'**un stimulant à base de sel biliaire ou cholérétique est co-administré.

8. Utilisation de β-cryproxanthine dans la fabrication d'un médicament pour le traitement d'une affection indésirable, **caractérisée en ce que** l'affection indésirable est choisie dans le groupe constitué de :
une maladie chronique, une cachexie tumorale, un trouble de l'alimentation, une infection virale, après une brûlure au deuxième ou troisième degré, après un infarctus du myocarde, après une transplantation d'organe, une immobilisation après une lésion squelettique ou une chirurgie, ou la prévention et/ou le traitement de la sarcopénie pendant le vieillissement par stimulation d'une formation de protéine augmentée et/ou prévention de la perte de protéine.
